# EUROPÄISCHE PATENTSCHRIFT

(11) **EP 1 356 845 B1**
(45) Veröffentlichungstag und Bekanntmachung des Hinweises auf die Patenterteilung: **06.10.2004**
(21) Anmeldenummer: 03006624.5
(22) Anmeldetag: 25.03.2003
(51) Int. Cl.: A61M 25/00, A61M 25/01

(54) **Katheter zur kardiovaskulären Anwendung**
Catheter for cardiovascular use
Catheter cardio-vasculaires

(30) Priorität: 19.04.2002 DE 10217509
(43) Veröffentlichungstag der Anmeldung: 29.10.2003
(60) Teilanmeldung: 04012667.4
(73) Patentinhaber: BIOTRONIK Mess- und Therapiegeräte GmbH & Co Ingenieurbüro Berlin, 12359 Berlin (DE)
(72) Erfinder: Kolberg, Gernot, 12043 Berlin (DE); Kranz, Curt, Dr., 14163 Berlin (DE); Flach, Erhard, 12305 Berlin (DE)
(74) Vertreter: Hübner, Gerd, Dipl.-Phys.

(56) Entgegenhaltungen:
- DE-A- 19 930 266
- US-A- 5 167 637
- US-A- 5 228 452
- US-A- 5 356 381
- US-A- 6 024 729

## Beschreibung

Die Erfindung betrifft einen Katheter zur kardiovaskulären Anwendung mit den im Oberbegriff des Anspruchs 1 angegebenen Merkmalen.

Kardiovaskulär anwendbare Katheter umfassen beispielsweise auch Elektroden für Herzschrittmacher oder Defibrillatoren, für die unterschiedlichste Ausführungsformen bekannt sind. Eine besondere Problematik bei solchen Elektroden tritt dann auf, wenn diese nicht nur in den leicht anzusteuernden Atrien oder Ventrikeln des Herzens positioniert, sondern in schwer zugänglichen Herzgefäßen, wie zum Beispiel dem Koronarsinus, eingeführt werden sollen.

Ein Katheter, der zum Einführen in den Koronarsinus oder andere schwer zugängliche Blutgefäße insbesondere bei Einsatz als Herzschrittmacherelektrodensonde geeignet ist, ist aus der DE 199 30 266 A1 bekannt. Dieser Katheter weist einen langgestreckten Katheterschaft mit einem Lumen auf, in dem ein hülsenartiger Mandrin - eine federelastische, relativ formstabile Führungshülse - verläuft. Dieser Mandrin wiederum nimmt einen Führungsdraht auf, der durch eine Schleusenöffnung am distalen Ende des Katheters daraus herausführbar ist.

Dieser Führungsdraht ist vorgebogen und wird als Sonde verwendet, die über die Katheterspitze hinaus vorgeschoben werden kann und aufgrund der Vorbiegung des Drahtes in eine von der Geradeaus-Richtung des Katheters abweisende Richtung weist. Auf diese Weise ist es möglich, mit dem vorgebogenen Draht in eine Abzweigung der Blutgefäße vorzudringen und den dann von dem vorgebogenen Draht geführten Katheter nachzuschieben.

Trotz der erhöhten Flexibilität und damit genaueren Steuerbarkeit dieses Katheters ist er in mehreren Punkten verbesserungsbedürftig. So ist der Katheter lediglich dafür vorgesehen, mit Mandrinhülse und Führungsdraht in das Herz eingeführt zu werden, wobei lediglich an kritischen Punkten der Führungsdraht durch Ausschieben und Lenken in beispielsweise ein Herzgefäß zum Einsatz kommt. Bei einem getrennten Einführen des Führungsdrahtes von außen und einem Nachschieben des Katheters über die gesamte Länge des Führungsdrahtes nach der sogenannten "Over-the-Wire-Technik" besteht das Problem, dass durch die Schleusendichtung an der distalen Durchtrittsöffnung für den Führungsdraht dieser einer hohen Reibung unterliegt, was zu einer Behinderung beim Aufschieben des Katheters auf den Führungsdraht führt.

Zum anderen ist der erörterte Katheter nur zum Einsatz mit Führungsdraht gedacht, während für Katheter zum Einsatz nur mit Mandrin andere Ausführungsformen gewählt werden. Insoweit werden im Stand der Technik grundsätzlich Katheter zur Verfügung gestellt, die entweder zum Einsatz mit Führungsdraht oder mit Mandrin konzipiert sind. Dies bedingt eine Verdoppelung des Herstellungs- und Lagerhaltungsaufwandes bei im Übrigen oft baugleichen Kathetern. Der entsprechende Mehraufwand schlägt sich auch bei der Logistik und Lagerhaltung in der klinischen Praxis nieder.

Ausgehend von den geschilderten Problemen des Standes der Technik, liegt der Erfindung die Aufgabe zugrunde, einen Katheter der eingangs geschilderten Art so weiterzubilden, dass er einerseits universell mit Führungsdraht oder Mandrin eingesetzt und andererseits bei Verwendung mit Führungsdraht ein leichtgängiges "Over-the-Wire-Schieben" des Katheters über den Führungsdraht erreicht werden kann.

Diese Aufgabe wird durch die im Kennzeichnungsteil des Anspruches 1 angegebenen Merkmale gelöst, wonach eine proximal vor der distalen Schleusenöffnung angeordnete Dichteinheit zur Abdichtung des Lumens vorgesehen ist, die von einer auswechselbaren Mandrinhülse reversibel durchstoßbar ist.

Durch die Auswechselbarkeit der Mandrinhülse kann der erfindungsgemäße Katheter zur Verwendung ohne Führungsdraht mit einem entsprechend ausgelegten Mandrin bestückt werden. Soll der Katheter dagegen mit einem Führungsdraht eingesetzt werden, so wird die ein Lumen für den Führungsdraht aufweisende Mandrinhülse verwendet, die wiederum - im Gegensatz zum eingangs genannten Stand der Technik - die Dichteinheit durchstößt. Damit wird das eigentliche Lumen des Katheters gegen Bluteintritt mithilfe der Mandrinhülse und der Dichteinheit als Dichtpartner gesperrt. Gleichzeitig kann jedoch der Führungsdraht im Lumen der Mandrinhülse ungehindert geschoben werden.

Im Übrigen wird darauf hingewiesen, dass als Mandrinhülse auch ein Schlauch oder anderweitiges röhrenförmiges Objekt im erfindungsgemäßen Sinn verwendbar ist.

Bevorzugtermaßen ist zwischen Dichteinheit und Schleusenöffnung ein Anschlag für die Durchstoßbewegung der Mandrinhülse vorgesehen. Dieser Anschlag verhindert zum einen, dass die Mandrinhülse versehentlich aus dem Katheter ausgeschoben wird und Herzmuskel- oder Gefäßwand-Gewebe in Mitleidenschaft zieht. Zum anderen dient dieser Anschlag als Widerlager für die Mandrinhülse, die so in herkömmlicher Weise für eine Streckung des Katheterschaftes im Bereich einer vorgeformten Schaftauslenkung dienen kann.

Gemäß einer weiteren bevorzugten Ausführungsform ist ein zweiter Anschlag proximal vor der Dichteinheit angeordnet, der mit einem Gegenstück an einem in das Lumen einführbaren Mandrin kooperiert. Dieser Anschlag dient wiederum einerseits als Widerlager für den Mandrin, andererseits verhindert er, dass der Mandrin die Dichteinheit beim Arbeiten ohne Führungsdraht durchstößt. Damit wird eine zuverlässige Abdichtung des Katheterlumens auch für diesen Anwendungsfall gewährleistet.

Bevorzugte Ausführungsformen der Erfindung betreffen ferner die Ausbildung der Anschläge als ringschulterförmige Durchmesserstufen und das Zusammenwirken des proximalen Anschlages mit einer Kugel am Gegenstück des führungsdrahtlosen Mandrins.

Durch die ferner vorzugsweise vorgesehene Isolierung von Mandrin beziehungsweise Mandrinhülse und Führungsdraht von den elektrischen Anschlussleitungen für die Katheterelektroden kann ein potentialfreier Führungsdraht erreicht werden. Dadurch können Funktionsstörungen der Katheterelektroden durch eine elektrische Verbindung mit dem im Blutstrom liegenden Führungsdraht vermieden werden.

Weitere Merkmale, Einzelheiten und Vorteile der Erfindung ergeben sich aus der nachfolgenden Beschreibung, in der ein Ausführungsbeispiel des Erfindungsgegenstandes anhand der beigefügten Zeichnungen näher erläutert wird. Es zeigen:
- Fig. 1: einen Axialschnitt durch das distale Ende eines Katheters mit eingesetztem Führungsdraht, und
- Fig. 2: einen Teilschnitt analog Fig. 1 mit einem eingesetzten Mandrin ohne Führungsdraht.

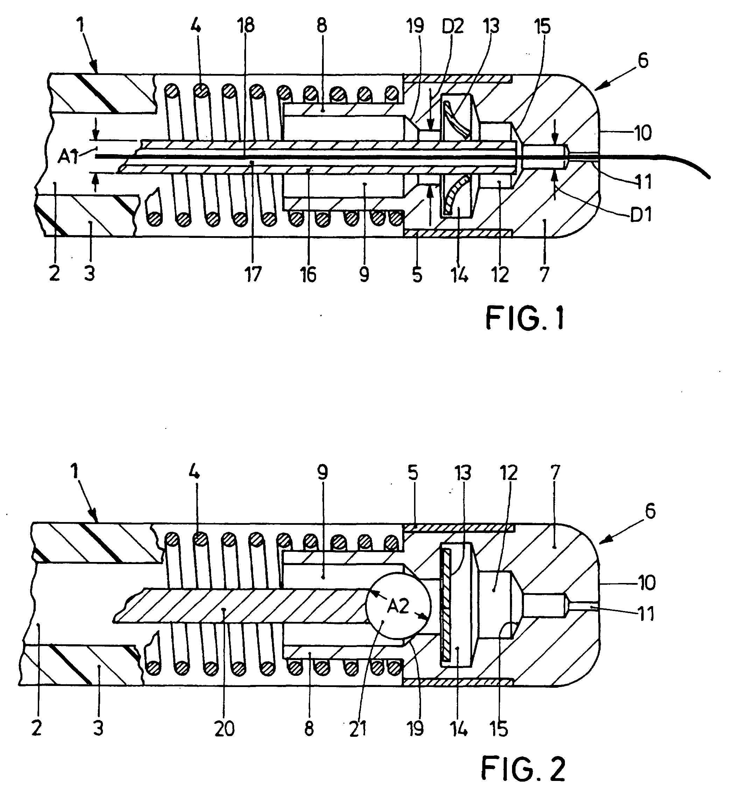

Der in Fig. 1 und 2 ausschnittsweise mit seinem distalen Ende gezeigte Katheter weist einen Katheterschaft 1 auf, in dem ein axial durchgehendes, zentrales Lumen 2 angelegt ist. In der Wand 3 des Katheterschaftes läuft eine gewendelte Anschlussleitung 4 für eine Ringelektrode 5, die in einem das distale Ende 6 des Katheters bildenden, isolierenden Kopfkörper 7 angeordnet ist.

Der Kopfkörper 7 weist in proximaler Richtung einen Ringansatz 8 auf, auf dem einerseits die gewendelte Anschlussleitung 4 mit ihrem distalen Ende sitzt und in dem andererseits das zentrale Lumen 2 durch die Innenöffnung 9 fortgesetzt ist.

In der distal weisenden Stirnfläche 10 des Kopfkörpers 7 ist zentral eine Schleusenöffnung 11 eingebracht, die über einen zentralen Zugangskanal 12 im Kopfkörper 7 vom Lumen 2 und der Innenöffnung 9 her zugänglich ist. Proximal vor der Schleusenöffnung 11 ist in diesem Zugangskanal 12 eine als flexible Scheibe mit einer zentralen, expandierbaren Öffnung ausgebildete Dichteinheit 13 angeordnet, die in einem Ringraum 14 des Zugangskanals 12 liegt. Zwischen Dichteinheit 13 und Schleusenöffnung 11 ist im Zugangskanal 12 ein erster, distaler Anschlag 15 vorgesehen, der durch eine entsprechende Durchmesserstufe gebildet ist. Der Zugangskanal 12 als Fortsetzung des Lumens 2 verringert sich dort auf einen Innendurchmesser D1, der kleiner ist, als der Außendurchmesser A1 einer Mandrinhülse 16, die im Lumen 2 verläuft, die Dichteinheit 13 durchstößt und durch die vorstehenden Dimensionsverhältnisse am distalen Anschlag 15 gegen ein weiteres Ausschieben aus dem Lumen 2 blockiert ist.

In der zentralen Hülsenöffnung 17 ist schließlich ein Führungsdraht 18 quasi frei und ohne relevante Reibung relativ zur Mandrinhülse 16 und damit zum Katheterschaft 1 verschiebbar. Insoweit kann der Katheter nach Einführen des Führungsdrahtes 18 in das gewünschte Herzgefäß problemlos über den Führungsdraht 18 geschoben werden. Durch die Dichteinheit 13 ist das Lumen 2 gegen eintretendes Blut sauber abgedichtet.

Wie ferner aus den beiden Fig. 1 und 2 hervorgeht, ist an der Basis des Ringansatzes 8 ein zweiter, proximaler Anschlag 19 in Form einer Durchmesserstufe vorgesehen, dessen Innendurchmesser D2 deutlich größer als der Innendurchmesser D1 des ersten, distalen Anschlages 15 ist. Insbesondere ist dieser Innendurchmesser D2 größer als der Außendurchmesser A1 der Mandrinhülse 16, sodass der Anschlag 19 ein Durchschieben der Mandrinhülse 16 durch die Dichteinheit 13 bis zum distalen Anschlag 15 nicht behindert.

Soll nun mit einem herkömmlichen Mandrin ohne Führungsdraht 18 gearbeitet werden, so kann die Mandrinhülse 16 entfernt und ein in Fig. 2 erkennbarer Mandrin 20 eingeschoben werden, der über einen Kugelkopf 21 an seinem distalen Ende als Gegenstück gegen den Anschlag 19 fährt. Der Außendurchmesser A2 des Kugelkopfes 21 ist größer als der Innendurchmesser D2 des proximalen Anschlags 19. Dadurch wird verhindert, dass der Mandrin 20 durch die Dichteinheit 13 hindurchgeschoben wird. Das Lumen 2 bleibt sauber abgedichtet.

Durch die Einbettung der Anschlussleitung 4 in die Wand 3 des Katheterschaftes 2 sind der Mandrin 20 beziehungsweise die Mandrinhülse 16 und der Führungsdraht 18 von der Anschlussleitung 4 elektrisch isoliert.

## Patentansprüche

1. Katheter zur kardiovaskulären Anwendung umfassend
- einen langgestreckten Katheterschaft (1),
- ein Lumen (2) im Katheterschaft (1),
- eine im Lumen (2) verlaufende Mandrinhülse (16), und
- einen in der Mandrinhülse (16) verlaufenden Führungsdraht (18), der durch eine Schleusenöffnung (11) am distalen Ende (6) des Katheters daraus herausführbar ist,
**gekennzeichnet durch**
- eine proximal vor der Schleusenöffnung (11) angeordnete, von der auswechselbaren Mandrinhülse (16) reversibel durchstoßbare Dichteinheit (13) zur Abdichtung des Lumens (2).

2. Katheter nach Anspruch 1, **dadurch gekennzeichnet, dass** im Lumen (2) zwischen Dichteinheit (13) und Schleusenöffnung (11) ein Anschlag (15) für die Durchstoßbewegung der Mandrinhülse (16) angeordnet ist.

3. Katheter nach Anspruch 1 oder 2, **dadurch gekennzeichnet, dass** im Lumen (2) ein zweiter Anschlag (19) proximal vor der Dichteinheit (13) angeordnet ist, der mit einem Gegenstück (21) an einem in das Lumen (2) einführbaren Mandrin (20) kooperiert.

4. Katheter nach Anspruch 2 und 3, **dadurch gekennzeichnet, dass** das der erste, distale und der zweite, proximale Anschlag (15, 19) durch ringschulterförmige Durchmesserstufen gebildet sind, wobei am ersten, distalen Anschlag (15) der Innendurchmesser (D1) des Lumens (2) geringer als am zweiten, proximalen Anschlag (19) ist.

5. Katheter nach Anspruch 3 oder 4, **dadurch gekennzeichnet, dass** das Gegenstück am Mandrin (20) eine Kugel (21) ist, deren Durchmesser (A2) größer als der Innendurchmesser (D2) des proximalen Anschlags (19) ist.

6. Katheter nach einem der vorgenannten Ansprüche, **dadurch gekennzeichnet, dass** der Mandrin (20), die Mandrinhülse (16) und der Führungsdraht (18) von den elektrischen Anschlussleitungen (4) für die Katheterelektroden (5) elektrisch isoliert sind.

## Claims

1. A catheter for cardiovascular application, comprising
- an elongated catheter shank (1);
- a lumen (2) inside the catheter shank (1);
- a mandrel sleeve (16) which extends in the lumen (2); and
- a guide wire (18) which runs in the mandrel sleeve (16) and is extendable through a sluice gate (11) out of the catheter at the distal end (6) thereof;
**characterized by**
- a sealing unit (13) for sealing the lumen (2), the sealing unit (13) being disposed proximally in front of the sluice gate (11) and reversibly pierced by the replaceable mandrel sleeve (16).

2. A catheter according to claim 1, **characterized in that** a stop (15) for the piercing motion of the mandrel sleeve (16) is disposed in the lumen (2) between the sealing unit (13) and the sluice gate (11).

3. A catheter according to claim 1 or 2, **characterized in that** a second stop (19) is disposed proximally in front of the sealing unit (13) in the lumen (2), the second stop (19) cooperating with a counterpart (21) on a mandrel (20) which is insertable into the lumen (2).

4. A catheter according to claim 2 and 3, **characterized in that** the first distal and the second proximal stop (15, 19) are formed by graduations in diameter in the form of annular shoulders, the inside diameter (D1) of the lumen (2) at the first distal stop (15) being less than at the second proximal stop ( 19).

5. A catheter according to claim 3 or 4, **characterized in that** the counterpart on the mandrel (20) is a ball (21), the diameter (A2) of which is greater than the inside diameter (D2) of the proximal stop (19).

6. A catheter according to one of the preceding claims, **characterized in that** the mandrel (20), the mandrel sleeve (16) and the guide wire (18) are electrically insulated towards the electric connecting lines (4) for the catheter electrodes (5).

## Revendications

1. Cathéter destiné à une utilisation cardiovasculaire, et comprenant :
- une tige de cathéter (1) très allongée ;
- une lumière intérieure (2), située dans la tige de cathéter (1) ;
- une douille formant mandrin (16), qui s'étend dans la lumière intérieure (2) ; et
- un fil de guidage (18), qui s'étend dans la douille formant mandrin (16) et qui peut sortir par un orifice formant passage étroit de tête (11) situé au niveau de l'extrémité distale (6) du cathéter,
**caractérisé par**
- une unité d'étanchéité (13), disposée de manière proximale avant l'orifice formant passage étroit de tête (11) et qui peut être traversée, de manière réversible, par la douille formant mandrin (16) échangeable, pour rendre étanche la lumière intérieure (2).

2. Cathéter selon la revendication 1, **caractérisé en ce qu'**il est prévu, dans la lumière intérieure (2) entre l'unité d'étanchéité (13) et l'orifice formant passage étroit de tête (11), une butée (15) pour le mouvement de traversée de la douille formant mandrin (16).

3. Cathéter selon la revendication 1 ou 2, **caractérisé en ce qu'**une seconde butée (19) est disposée dans la lumière intérieure (2) de manière proximale avant l'unité d'étanchéité (13) et **en ce qu'**elle coopère avec une pièce formant pendant (21), située au niveau d'un mandrin (20) propre à être introduit dans la lumière intérieure (2).

4. Cathéter selon les revendications 2 et 3, **caractérisé en ce que** la première butée (15) distale et la seconde butée (19) proximale sont constituées par des gradins du diamètre sous la forme d'épaulements annulaires, le diamètre intérieur (D1) de la lumière intérieure (2) au niveau de la première butée (15) distale étant plus petit qu'au niveau de la seconde butée (19) proximale.

5. Cathéter selon la revendication 3 ou 4, **caractérisé en ce que** la pièce formant pendant, prévue sur le mandrin (20), est un élément sphérique (21) dont le diamètre (A2) est plus grand que le diamètre intérieur (D2) de la butée (19) proximale.

6. Cathéter selon l'une des revendications précédentes, **caractérisé en ce que** le mandrin (20), la douille formant mandrin (16) et le fil de guidage (18) sont électriquement isolés par rapport aux lignes de raccordement électrique (4) pour les électrodes de cathéter (5).
